# EUROPÄISCHE PATENTSCHRIFT

(11) **EP 1 200 521 B1**
(45) Veröffentlichungstag und Bekanntmachung des Hinweises auf die Patenterteilung: **10.11.2004**
(21) Anmeldenummer: 01902385.2
(22) Anmeldetag: 02.02.2001
(51) Int. Cl.: C08L 33/06, A61K 9/20, A61K 9/32

(54) **DISPERSION MIT NICHTIONISCHEM EMULGATOR**
DISPERSION COMPRISING A NON-IONIC EMULSIFIER
DISPERSION CONTENANT UN EMULSIFIANT NON IONIQUE

(30) Priorität: 10.03.2000 DE 10011447
(43) Veröffentlichungstag der Anmeldung: 02.05.2002
(73) Patentinhaber: Röhm GmbH & Co. KG, 64293 Darmstadt (DE)
(72) Erfinder: MEIER, Christian, 64295 Darmstadt (DE); EISELE, Johanna, 64291 Darmstadt (DE); SCHNABEL, Michael, 64584 Biebesheim (DE); SCHULTES, Klaus, 65197 Wiesbaden (DE); GRIMM, Stefan, 67547 Worms (DE); PETEREIT, Hans-Ulrich, 64291 Darmstadt (DE); SÜFKE, Thomas, 64390 Erzhausen (DE)
(86) Internationale Anmeldenummer: PCT/EP2001/001108
(87) Internationale Veröffentlichungsnummer: WO 2001/068767

(56) Entgegenhaltungen:
- EP-A- 0 315 218
- DE-A- 19 918 435
- JP-A- 1 113 322
- GÖPFERICH, A. ET AL.: "The influence of endogenous surfactant on the structure and drug-release properties of Eudragit NE30D-matrices" J. OF CONTROLLED RELEASE, Bd. 18, Nr. 2, 1992, Seiten 133-144, XP000247010 Amsterdam in der Anmeldung erwähnt

## Beschreibung

Die Erfindung betrifft das Gebiet der Dispersionen und deren Verwendung als Überzugs- und Bindemittel für Arzneiformen.

### Stand der Technik

Die Verwendung von sogenannten neutralen Methacrylat-Copolymeren, das heißt Methacrylat-Copolymeren, die zum überwiegenden Teil aus (Meth)acrylat-Monomeren mit neutralen Resten, wie Methylmethacrylat oder Ethylacrylat, bestehen, als Überzugs- und Bindemittel für Arzneiformen mit verzögerter Wirkstofffreisetzung ist seit langem bekannt.

Verwendungen in Mischungen mit anionischen Dispersionen sind beschrieben z. B. in EP-A 152 038, EP-A 208 213 oder EP-A 617 972.

Die neutralen Methacrylat-Copolymere werden heutzutage bevorzugt als Dispersionen verwendet. Derartige Dispersionen werden durch Emulsionspolymerisation hergestellt und enthalten deshalb einen herstellungsbedingt einen Emulgator, der auch die Stabiltät der erhaltenen Dispersion als solche bewirkt. In der fertigen Arzneiform beeinflußt der enthaltene Emulgator zudem die Wirkstofffreisetzungscharakteristik.

Durch den Verwendungszweck in Arzneimitteln und da die Copolymere aufgrund der Monomerzusammensetzung keine oder nur geringe Ladungen aufweisen, ist die Auswahl geeigneter Emulgatoren sehr eingeschränkt.

Göpferich und Lee beschreiben in *"The influence of endogenous surfactant on the structure and drug-release properties of Eudragit NE30D-matrices*", Journal of Controlled Release 18 (1992), S. 133 - 144, daß ein in der Dispersion enthaltener Emulgator vom Typ Nonylphenol Probleme bei der Wirkstoff-freisetzung von überzogenen Arzneiformen verursacht. Die Autoren beschreiben eine anisotrope Struktur in aus der Dispersion erhaltenen Copolymer-Filmen. Dabei kommt es sowohl in wirkstofffreien und auch in wirkstoffhaltigen Filmen in Abhängigkeit von der Lagerungsdauer und dem Wirkstoffgehalt zu einer Phasenseparation und zu einer Kristallisation des Emulgators. Diese bewirken offensichtlich Ungleichmäßigkeiten bei der Freisetzung des Wirkstoffs Clenbuterol. Entfernt man den Emulgator aus gefriergetrocknetem Copolymer durch Auswaschen mit Wasser, so wird bei dem gereinigten Copolymer eine gleichmäßige wenn auch verlangsamte Wirkstofffreisetzung beobachtet.

DE-A 195 03 099 beschreibt ein Verfahren zur Herstellung wäßriger Polymerisatdispersionen nach der Methode der radikalischen wäßrigen Emulsionspolymerisation in Gegenwart eines nichtionischen Emulgators. Als nichtionische Emulgatoren sind solche geeignet, deren Trübungspunkt unterhalb der Polymerisationstemperatur liegt. Eine Vielzahl geeigneter Verbindungen wird aufgeführt, u.a. auch Nonylphenol-Emulgatoren.

JP 01 113322 A beschreibt ein Emulsionspolymerisationsverfahren zur Herstellung von (Meth)acrylatcopolymeren, wobei Natriumdodecylsulfat (SDS) als Emulgator eingesetzt wird.

DE 199 18 435 A1 beschreibt ein Verfahren zur Herstellung von (Meth)acrylatcopolymeren mit tertiären Aminogruppen, wobei Emulgatoren mit HLB-Werten > 14 Anwendung finden.

### Aufgabe und Lösung

Es wurde als Aufgabe gesehen, Dispersionen des Standes der Technik , enthaltend Methacrylat-Copolymere.mit geringen oder keinen Anteilen an , Monomeren mit ionischen Resten dahingehend zu verbessern, daß unter Beibehaltung der Stabilität der Dispersion und ihrer Teilchengrößenverteilung, sich daraus Arzneimittelformulierungen herstellen lassen, bei denen eine Phasenseparation unter Ausbildung von Kristallstrukturen durch den Emulgator unterbleibt. Dabei sollen die Wirkstofffreisetzungscharakteristik und sonstige z. B. mechanische Eigenschaften nicht zum Nachteil verändert werden.

### Die Aufgabe wurde gelöst durch eine

Dispersion, geeignet zur Verwendung als Überzugs- und Bindemittel für Arzneiformen, mit einem Feststoffgehalt von 10 - 70 Gew.-%, enthaltend
a) 90 bis 99 Gew.-% eines Methacrylat-Copolymeren, das zu mindestens zu 90 Gew.-% aus (Meth)acrylat-Monomeren mit neutralen Resten besteht und eine Glastemperatur Tg von - 20 °C bis + 20 °C bestimmt nach der DSC-Methode, und
b) einen Emulgator,
dadurch gekennzeichnet, daß
als Emulgator b) 1 - 10 Gew.-% eines nichtionischen Emulgators mit einem HLB-Wert von 15, 2 bis 17,3 enthalten sind.

### Ausführung der Erfindung

### Methacrylat-Copolymer

Die erfindungsgemäße Dispersion enthält 90 - 99 Gew.-% bezogen auf den Feststoffanteil eines Methacrylat-Copolymers.

Das Methacrylat-Copolymer besteht mindestens zu 90, insbesondere 95, bevorzugt zu 97, insbesondere zu 99, besonders bevorzugt zu 100 Gew.-% aus (Meth)acrylat-Monomeren mit neutralen Resten, insbesondere C₁- bid C₄-Alkylresten.

Geeignete Monomere sind z. B. Methylmethacrylat, Ethylmethacrylat, Butylmethacrylat, Methylacrylat, Ethylacrylat, Butylacrylat. Bevorzugt sind Methylmethacrylat, Ethylacrylat und Methylacrylat.

In geringen Anteilen, zu höchstens 10, bevorzugt höchstens 5, besonders bevorzugt höchstens 3 oder höchstens 1 Gew.-% können Methacrylatmonomere mit anionischen Resten, z. B. Methacrylsäure, enthalten sein.

Das Methacrylat-Copolymer weist eine Glastemperatur Tg von - 25 °C bis + 20 °C, bevorzugt - 10 °C bis 0 °C, bestimmt nach der DSC-Methode (ISO 11357) auf.

Ein typisches Methacrylat-Copolymer kann z. B. aus 25 - 35 Gew.-% Methylmethacrylat und 75 bis 65 Gew.-% Ethylacrylat aufgebaut sein.

Erfindungsgemäß können die an sich neutralen Polymere geringe Mengen Methacrylsäure enthalten, die zwar die Wasserunlöslichkeit des Polymeren praktisch nicht verändern, jedoch die Quellung beeinflußen können und eine pH-abhängige Steuerung der Permeabilität erlauben.

### Emulgatoren

Die erfindungsgemäße Dispersion enthält 1 bis 10, bevorzugt 2 bis 8, besonders bevorzugt 4 bis 6 Gew.-% bezogen auf den Feststoffanteil eines nichtionischen Emulgators mit einem HLB-Wert von 15, 7 bis 16,2.

Emulgatoren kontrollieren den Ablauf des Emulsionspolymerisationsverfahrens, im dem sie die kettenaufbauende Reaktion der emulgierten Monomere in der Wasserphase ermöglichen. Sie sind daher ein für die Herstellung notwendiger Hilfsstoff und bestimmend für die Eigenschaften der Dispersion. Sie können üblicherweise nicht ausgetauscht werden, ohne relevante Eigenschaften der Dispersion grundlegend zu verändern.

Der HLB-Wert ist ein 1950 von Griffin eingeführtes Maß der Hydrophilie bzw. Lipophilie von nichtionischen Tensiden. Er läßt sich experimentell durch die Phenol-Titrationsmethode nach Marszall bestimmen; vgl. "Parfümerie, Kosmetik", Band 60, 1979, S. 444 - 448; weitere Literaturhinweise in Römpp, Chemie-Lexikon, 8.Aufl. 1983, S.1750. Siehe weiterhin z. B. US 4 795 643 (Seth)).

Ein HLB-Wert (Hydrophile/Lipophile Balance) läßt sich nur bei nicht ionischen Emulgatoren exakt bestimmen. Bei anionischen Emulgatoren kann dieser Wert rechnerisch ermittelt werden, liegt jedoch praktisch immer über oder weit über 20.

Die HLB-Werte der Emulgatoren haben einen deutlichen Einfluß auf die Auskristallisation des Emulgators. Im Idealfall liegen diese Werte zwischen 15, 7 und 16,2. Oberhalb des beanspruchten Bereiches kristallisieren die Emulgatoren nach dem Trocknen aus. Emulgatoren mit einem HLB-Wert unterhalb des beanspruchten Bereiches können die Dispersion nicht ausreichend stabilisieren was an starker Koagulatbildung zu erkennen ist. Die HLB Werte wurden entweder der Literatur (Fiedler: Lexikon der Hilfsstoffe) entnommen bzw. nach W. C. Griffin (Sonderdruck aus Parfümerie und Kosmetik 64, 311-314, 316 (1983); Hüthig Verlag, Heidelberg / Pharmind Ind. 60 Nr.1 (1998); Dielektrizitätsthermoanalyse) berechnet.

Der Emulgator soll toxikologisch unbedenklich und daher bevorzugt nichtionisch Emulgatoren sein.

Geeignete Emulgatorklassen sind ethoxylierte Fettsäureester oder -ether, ethoxylierte Sorbitanether, ethoxylierte Alkylphenole, Glycerin- oder Zuckerester oder Wachsderivate

Geeignete Emulgatoren sind zum Beispiel Polyoxyethylenglycerinmonolaurat, Polyoxyethylenglycerinmonostearat, Polyoxyethylen-20-cetylstearat Polyoxyethylen-25-cetylstearat, Polyoxyethylen(25)oxypropylenmcinostearat, Polyoxyethylen-20-sorbitanmonopalmitat, Polyoxyethylen-16-tert.oktylphenol, Polyoxyethylen-20-cetylether, Polyethylenglykol(1000)monocetylether, ethoxyliertes Rizinusöl, Polyoxyethylensorbitol-Wollwachs-Derivate, Polyoxyethylen(25)propylenglykolstearat und Polyoxyethylensorbitester

Bevorzugt sind Polyoxyethylen-25-cetylstearat, Polyoxyethylen-20-sorbitanmonopalmitat, Polyoxyethylen-16-tert.oktylphenol und Polyoxyethylen-20-cetylether.

### Herstellung der Dispersion

Die neue Dispersion wird in an sich bekannter Weise durch wäßrige Emulsionspolymerisation im Batch- oder im Zulaufverfahren, halbkontinuierlich oder auch kontinuierlich gewonnen (s. dazu z.B. DE 195 03 099 A1).

Die radikalische Polymerisation der Monomeren in Gegenwart des Emulgators erfolgt mittels radikalbildender wasserlöslicher Polymerisationsinitiatoren, bei denen die Radikalbildung thermisch oder über Redoxprozesse erfolgen kann. Gegebenenfalls werden Molekulargewichts-Regler zur Einstellung der Molmassen zugesetzt. Emulsionspolymerisate werden üblicherweise in Konzentrationen zwischen 10 und 70 Gew.% hergestellt. Günstig ist ein Feststoffgehalt von 30 - 50 Gew.%. Die diskontinuierliche Herstellung erfolgt in der Regel in Rührkessel-Reaktoren.

Zur Herstellung werden bei einer einfachen Batchherstellung alle Monomeren gemäß der gewünschten Copolymerzusammensetzung zusammen mit dem Emulgator, Initiatoren, Reglern und sonstigen Hilfsmitteln zusammen mit Wasser in einem Reaktionskessel vorgelegt und darin gelöst bzw. dispergiert. Durch Aktivierung des Starters (Erhöhung der Temperatur, Zugabe des Redoxmittels) wird die polymere Kettenreaktion initiiert und durchgeführt. Hierbei bilden sich die bekannten aus Polymerketten bestehenden Latexteilchen aus.

Der Dispersion können Antischaumemulsion und Stabilisatoren zugegeben werden.

### Verwendungen

Die neuen Überzugsmittel können in entsprechender Weise wie andere bekannte wäßrige Überzugsmittel auf Acrylatbasis verarbeitet werden. Am gebräuchlichsten sind Überzüge auf 0,1 bis 3 mm große Partikel nach dem Wirbelschichtverfahren. Übliche Zusätze, wie Pigmente, Füllstoffe, Verdickungsmittel, Entschäumungsmittel, Konservierungsmittel usw. können in gebräuchlichen Mengen mitverwendet werden.
Überzüge können auf Tabletten, Kapseln, Dragees, Granulaten öder Kristallen erzeugt werden. Auch die Bildung von Matrix-Tabletten oder -Granulaten ist möglich. Bevorzugte Verarbeitungstemperaturen liegen im Bereich von 20 bis 40°C. Geeignete Filmdicken betragen 10 bis 80 Mikrometer.

Nach dem Mechanismus der Wirkstoffabgabe durch Diffusion kann mittels des Überzugsfilms nicht nur im Magen-Darm-Trakt, sondern auch in anderen Körperhöhlen, Geweben, Blutbahnen und den Lebensräumen von Tieren und Pflanzen genutzt werden, um dort im Zusammenhang verzögerte Freisetzung von Wirkstoffen herbeizuführen. Beispiele sind Filme, die mit Kathetern in die Blutbahn eingebracht werden, Implantate von Tierarzneimitteln.

Ebenso wie mit anderen wäßrigen Überzugsmitteln können Schichten von mehrlagigen Überzugssystemen erzeugt werden. Beispielsweise kann ein Kern, der z.B. basische oder wasserempfindliche Wirkstoffe enthält, mit einer Isolierschicht aus einem anderen Überzugsmaterial, wie Celluloseether, Celluloseester, kationische Polymethacrylate (wie EUDRAGIT® E 100, -RL 100, RS 100, Röhm GmbH), versehen werden, bevor das erfindungsgemäße Überzugsmittel aufgetragen wird. Ebenso können anschließend weitere Überzüge, beispielsweise mit geruchs- oder geschmackskaschierender Wirkung oder mit ansprechender Farb- oder Glanzwirkung, aufgebracht werden.

Die Freisetzungs-Charakteristik von Arzneimittelüberzügen in vitro wird nach USP üblicherweise mit künstlichem.Magensaft (0,1N HCl) und künstlichem Darmsaft (pH 6,8) geprüft.

### Weitere Anwendungen sind folgender Literatur beschrieben:

Bauer, Lehmann, Osterwald, Rothgang: Coated Dosage Forms, CRC Press LLC, Boca Raton, Florida, Medpharm Scientific Publishers, Stuttgart 1998
I. Ghebre-Sellassie, Multiparticulate Oral Drug Delivery, Marcel Dekker, Inc. New York, Basel, Hong Kong, 1994

### Sprühaufträge aus Mischungen mit anderen Dispersionen:

K. Lehmann, D. Dreher: Mixtures of Aqueous Polymethacrylate Dispersions for Drug Coating, Drugs made in Germany 31 101-102 (1988)

### Matrixtabletten über Feuchtgranulation

K. Lehmann, H.-U. Petereit, Verwendung wäßriger Poly(meth)acrylat-Dispersionen für die Herstellung von Matrixtabletten, Acta Pharm. Technol. 34(4)189-195 (1988)
J. McGinity, Aqueous Polymeric Coatings for Pharmaceutical Dosage Forms, 2^{nd} Edition, Marcel Dekker, Inc. New York, Basel, Hong Kong, 1996

### Zerfallende Retardtabletten

K. Lehmann, H.-U. Petereit, D. Dreher, Schnellzerfallende Tabletten mit gesteuerter Wirkstoffabgabe , Pharm. Ind. 55,(10) 940-947 (1993)
K. Lehmann, H.-U. Petereit, D. Dreher, Fast Disintegrating Controlled Release Tablets from Coated Particles, Drug Made in Germany 37(2), 53-60(1994)
R. Bodmeier, Tabletting of Coated Pellets Eur. J.Phar and Biopharm. 43 1-8(1997)

### (Trans)dermale Therapiesysteme

- Heilmann, K. : Therapeutische Systeme, Ferdinand Euler Verlag, Stuttgart, S. 52 -57.
- Brandau, R. und Lippold, B. H. (1982) : Dermal änd Transdermal Absorption. Wissenschaftliche Verlagsgesellschaft mbH, Stuttgart, S. 171 - 200.

H.-U. Petereit, 3^{rd} European Congress of Biopharmaceutics and Pharmacokinetics-Proceed. Vol. I, 84-93(1987)

### Verwendung des Feststoffs:

Der aus den erfindungsgemäßen Dispersionen durch Trocknung, Koagulation oder Abquetschextrusion gewonnene Feststoff kann folgendermaßen eingesetzt werden:
Extrusion: Ggf. nach Abmischen mit Hilfsstoffen und/oder Wirkstoffen zu Granulaten, Folien u. ä..
Spritzguß: Gemäß der neue Spriztgußanmeldung zu Hohlkörpern und monolithischen Trägern
Auflösung: Das Polymerisat ist in gebräuchlichen Lösungsmitteln wie kurzkettige Alkohole oder Ketonen löslich. Solche Lösungen lassen sich in übliche Beschichtungsverfahren einsetzen.

### Vorteilhafte Wirkungen der Erfindung

Die erfindungsgemäßen Dispersionen werden als Binde- und Bindemittel in der Arzneimittelherstellung eingesetzt. Primär müssen also die für diesen Effekt notwendigen physikalisch chemischen Eigenschaften erreicht werden (s. Beispiel 4 und 5). Insbesondere ist dabei eine sichere Filmbildung bei Temperaturen unter 10°C vorteilhaft, sodass eine Verarbeitung ohne Weichmacherzusatz möglich ist. Die reproduzierbare Koaleszenz der Latexpartikeln erlaubt die Formulierung von Retardarzneiformen.

Wenn die oben beschriebene Kristallisation von Emulgatoren auf tritt, stellt sie eine erhebliche Qualitätsminderung von Arzneimitteln dar. Im Sinne der Arzneimittelsicherheit sollte also die Auskristallisation der Emulatoren nach dem Trocknen verhindert werden. Dieser Effekt wird offensichtlich durch strukturbedingte Wechselwirkung des Emulgators mit dem Polymer erreicht. Die erfindungsgemäßen Dispersionen erlauben also die Entwicklung verläßlicherer Retardarzneiformen.

Die Erfindung eignet sich sich insbesondere für die Bereitstellung von Arzneiformen enthaltend die unten stehenden Wirkstoffe.

### Therapeutische Kategorien:

Analgetika, Antirheumatika, Antiallergika, Antiarrhythmika, Betarezeptorenblocker, Calciumkanalblocker, Hemmstoffe des Renin-Angiotensin-Systems, Broncholytika/Antiasthmatika Cholinergika Diuretika Durchblutungsfördernde Mittel Gichtmittel Grippemittel Koronarmittel Lipidsenker Magen-Darmmittel Psychopharmaka Thrombozytenaggregationshemmer Urologika Venetherapeutika Vitamine und Mineralien

### Wirkstoffe

Morphin und dessen Derivate, Tramadol, Acetylsalicylsäure, Diclofenac, Indometacin, Lonazolac, Ibuprofen, Ketoprofen, Propyphenazon, Naproxen, Paracetamol, Flurbiprofen, Dimetinden, Chinidin, Metoprolol, Propranolol, Oxprenolol, Pindolol, Atenolol, Metoprolol, Disopyramid, Verapamil, Diltiazem, Gallopamil, Nifedipin, Nicardipin, Nisoldipin, Nimodipin, Amlodipin, Theophyllin, Salbutamol, Terbutalin, Ambroxol, Aminophyllin, Cholintheophyllinat, Pyridostigmin, Piretanid, Furosemid, Pentoxifyllin, Naftidrofuryl, Buflomedil, Xantinolnicotinat, Bencyclan, Allopurinol, Norephedrin, Clorphenamin Isosorbidmononitrat, Isosorbiddinitrat, Glyceroltrinitrat, Molsidomin, Bezafibrat, Fenofibrat, Gemfibrozil, Cerivastatin, Pravastatin, Fluvastatin, Lovastatin, Atorvastatin, Simvastatin, Xantinol, Metoclopramid, Amitriptylin, Dibenzepin, Venlafaxin, Thioridazin, Oxazepam, Lithium, Nitrofurantoin, pflanzliche Trockenextrakt, Ascorbinsäure und Kalium und deren pharmazeutisch verwendete Salze.

### BEISPIELE

### Untersuchungsmethoden:

Feststoffgehalt: 1 g Dispersion wird in einem Ofen über 3 Stunden bei 110°C gemäß Pharm. Eur. 2.2.32 Methode d getrocknet.
pH-Wert: Bestimmt nach Pharm. Eur. Methode 2.2.3.

Dynamische Viskosität: Bestimmt mit einem Brookfield Viskosimeter (UL Adapter / 30 min⁻¹ / 20°C)
Teilchengröße: Bestimmt aus verdünnter Dispersion mit einem Nanosizer (Fa. Coulter).
Koagulatanteil: Über ein genau gewogenes Sieb mit 0.09 mm Maschenweite (mesh no. 90, ISO) werden 100 g Dispersion gegeben und mit gereinigtem Wasser nachgewaschen, bis der Durchlauf klar ist. Sieb und Rückstand werden bei 105°C bis zur Gewichtskonstanz getrocknet und genau gewogen. Die Gewichtsdifferenz wird als % der untersuchten Dispersionsmenge berechnet.

### Kristallisation des Emulgators:

Es werden ca. 0,3g Dispersion auf einen Objektträger gegeben und bei <10°C für mind. 12 h getrocknet. Die Kristallisation des Emulgators im getrockneten Film wird anschließend unter einem Polarisationsmikroskop bei 400-facher Vergrößerung überprüft. Die Kristallbildung ist an den farbigen Lichtdoppelbrechungen zu sehen, amorphe Bereiche sind dunkel erkennbar.

### 1. - 5. Variation der Polymerzusammensetzung

Zur Herstellung der Dispersion werden in einem Reaktionskessel 55,0kg Wasser vorgelegt und darin 328 g Polyoxyethylen-20-cetylether gelöst. Nach dem Lösevorgang werden die Monomere gemäß Tabelle16,6kg Ethylacrylat, 7,1kg Methylmethacrylat und 0,3kg Methacrylsäure zugegeben und bei 30°C emulgiert.
Zum Starten der Reaktion werden die wasserlöslichen Initiatoren (0,22g Eisen(II)-sulfat gelöst in 160g Wasser, 22,0g Ammoniumperoxodisulfat und 30,8g Natriumdisulfit, jeweils gelöst in 320g Wasser) zugegeben. Nach Erreichen der Temperaturspitze wird der Ansatz abgekühlt. Bei ca. 50°C werden 754g Emulgator gemäß Tabelle zur Nachstabilisierung zugegeben. Nach Erreichen von 40°C werden zur Nachreaktion 6,7g Ammoniumperoxodisulfat, gelöst in 160g Wasser zugesetzt und die Dispersion filtriert und anschließend desodoriert.

| Bsp. | Ethylacrylat [Teile] | Methymethacrylat [Teile] | Methacrylsäure [Teile] | Feststoffgehalt [%] | pH-Wert | dynamische Viskosität [mPa*s] | Teilchengrößer_{NS} [nm] | Kristallisation des Emulgarors |
|---|---|---|---|---|---|---|---|---|
| 1 | 70 | 30 | 0 | 30,6 | 2,7 | < 50 | 60,3 | nein |
| 2 | 69 | 30 | 1 | 30,9 | 8,3 | < 50 | 76,0 | nein |
| 3 | 68 | 29 | 3 | 30,6 | 2,8 | < 50 | 57,5 | nein |
| 4 | 66 | 29 | 5 | 30,7 | 2,7 | < 50 | 61,8 | nein |
| 5 | 63 | 27 | 10 | 30,3 | 2,6 | < 50 | 89,0 | nein |

### 6-15. Variation des Emulgators

Zur Herstellung der Dispersion werden in einem Reaktionskessel 55,0kg Wasser vorgelegt und darin 328g Emulgator gemäß Tabelle gelöst. Nach dem Lösevorgang werden 16,6kg Ethylacrylat, 7,1 kg Methylmethacrylat und 0,3kg Methacrylsäure zugegeben und bei 30°C emulgiert.
Zum Starten der Reaktion werden die wasserlöslichen Initiatoren (0,22g Eisen(II)-sulfat gelöst in 160g Wasser, 22,0g Ammoniumperoxodisulfat und 30,8g Natriumdisulfit, jeweils gelöst in 320g Wasser) zugegeben. Nach Erreichen der Temperaturspitze wird der Ansatz abgekühlt. Bei ca. 50°C werden 754g Emulgator gemäß Tabelle zur Nachstabilisierung zugegeben. Nach Erreichen von 40°C werden zur Nachreaktion 6,7g Ammoniumperoxodisulfat, gelöst in 160g Wasser zugesetzt und die Dispersion filtriert und anschließend desodoriert.

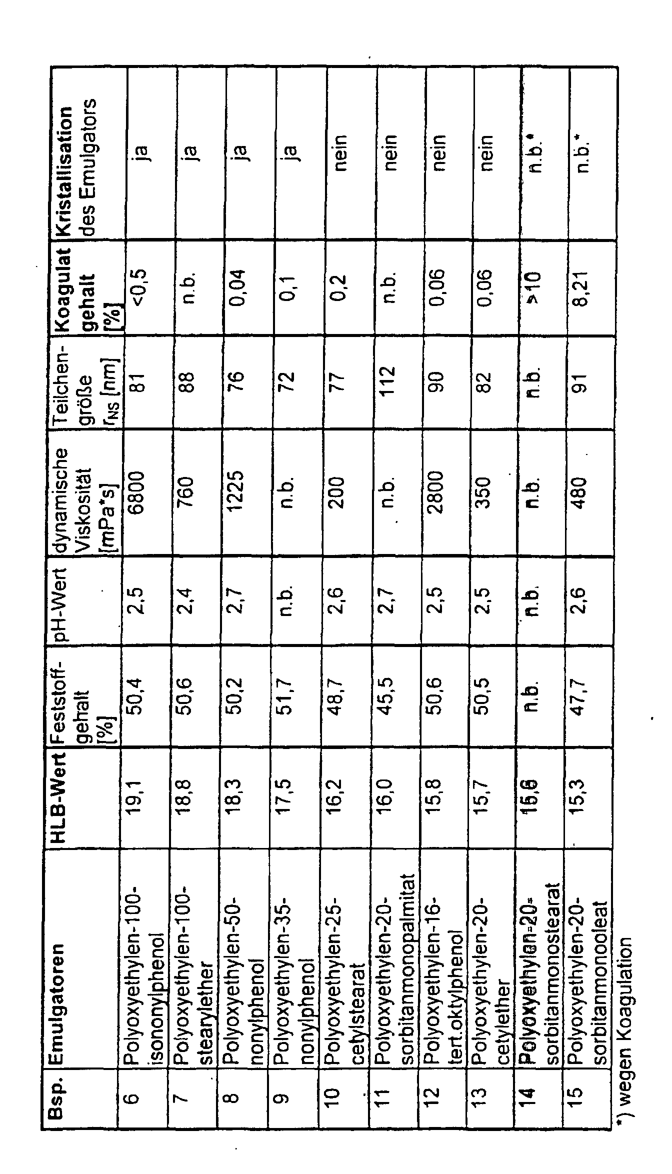

### 16.- 18 Variation des Herstellungsverfahrens

### 16. Dispersionsherstellung über Emulsionspolymerisation im Einstufenbatchverfahren

Zur Herstellung der Dispersion werden in einem Reaktionskessel 55,0kg Wasser vorgelegt und darin 328g Polyoxyethylen-20-cetylether gemäß Tabelle gelöst. Nach dem Lösevorgang werden 16,6kg Ethylacrylat, 7,1 kg Methylmethacrylat und 0,3kg Methacrylsäure zugegeben und bei 30°C emulgiert.
Zum Starten der Reaktion werden die wasserlöslichen Initiatoren (0,22g Eisen(II)-sulfat gelöst in 160g Wasser, 22,0g Ammoniumperoxodisulfat und 30,8g Natriumdisulfit, jeweils gelöst in 320g Wasser) zugegeben. Nach Erreichen der Temperaturspitze wird der Ansatz abgekühlt. Bei ca. 50°C werden 754g Emulgator gemäß Tabelle zur Nachstabilisierung zugegeben. Nach Erreichen von 40°C werden zur Nachreaktion 6,7g Ammoniumperoxodisulfat, gelöst in 160g Wasser zugesetzt und die Dispersion filtriert und anschließend desodoriert.

### 17. Dispersionsherstellung Dispersion über Emulsionspolymerisation in einem doppelten Batchverfahren

Für den 1. Batch der Dispersion werden in einem Reaktionskessel 23,0kg Wasser vorgelegt und darin 512g Emulgator gemäß Tabelle gelöst. Nach dem Lösevorgang werden 8,30kg Ethylacrylat, 3,55kg Methylmethacrylat und 0,14kg Methacrylsäure zugegeben und bei 30°C emulgiert.
Zum Starten der Reaktion werden die wasserlöslichen Initiatoren (0,22g Eisen(II)-sulfat, 11,0g Ammoniumperoxodisulfat und 15,4g Natriumdisulfit, jeweils gelöst in 160g Wasser) zugegeben. Nach Erreichen der Temperaturspitze wird der Ansatz auf 50°C abgekühlt.
Für den 2. Batch werden dem 1. Batch werden 570g Emulgator zugegeben und das Gemisch 30 Minuten gerührt. Anschließend wird analog dem 1. Batch die selbe Menge Monomere zugefügt, 10 Minuten gerührt und die Initiatoren (11,0g Ammoniumperoxodisulfat und 15,4g Natriumdisulfit, jeweils gelöst in 160g Wasser) zugegeben. Nach Reaktionsende wird der Ansatz auf 40°C abgekühlt und Initiator (6,7g Ammoniumperoxodisulfat, gelöst in 160g Wasser) zur Nachreaktion zugegeben. Zur Desodorierung wird die Dispersion in einem Reaktionskessel mit verdünnter Natronlauge auf einen pH-Wert von ca. 8 eingestellt und 10-15% des Dispersionswasser abdestilliert. Anschließend wird die Dispersion auf einen Feststoffgehalt von ca. 30% verdünnt. Danach wird die Dispersion filtriert.

### 18. Dispersionsherstellung Dispersion über Emulsionspolymerisation im Zulaufverfahren

In einem Glasreaktor werden 2370g Wasser und 5,0g Emulgator gemäß Tabelle unter Rühren auf 80°C erhitzt. In der Zwischenzeit wird eine Präemulsion bestehend aus 1800g Wasser, 64,9g Emulgator, 3,0g Ammoniumperoxodisulfat, 1245,6g Ethylacrylat, 532,8g Methylmethacrylat und 21,6g Methacrylsäure mit Hilfe eines hochscherenden Rührers hergestellt.. Die für den Start der Reaktion bereitgestellte Initiatormenge (1,2g Ammoniumperoxodisulfat, gelöst in 30g Wasser) wird der Vorlage zugesetzt und die Präemulsion innerhalb vier Stunden bei 80°C der Vorlage zudosiert. Nach Zulaufende wird die entstandene Dispersion noch zwei Stunden bei 80°C gerührt, anschließend auf Raumtemperatur abgekühlt und mit verdünnter Natronlauge auf einen pH-Wert von ca. 8 eingestellt und 10-15% des Dispersionswasser abdestilliert. Anschließend wird die Dispersion auf einen Feststoffgehalt von ca, 30% verdünnt. Anschließend wird die Dispersion filtriert.

Die erhaltenen Dispersion wurden auf die in der Tabelle angegebenen Eigenschaften geprüft. In der Tabelle sind die Analysenwerte der Dispersionen nach oben genannten Herstellungsbedingungen aufgeführt.

| Dispersion | Feststoffgehalt [%] | pH-Wert | dynamische Viskosität [mPa*s] | Teichengröße r_{NS} [nm] | Kristallisation des Emulgators |
|---|---|---|---|---|---|
| 16 | 30,9 | 8,3 | <10 | 76 | nein |
| 17 | 29,6 | 8,3 | <10 | 78 | nein |
| 18 | 30,2 | 8,3 | <10 | 90 | nein |

### 19. Verwendung der Dispersion als Überzugsmittel:

### a) Überzüge auf Kaliumchloridkristalle.

In einem Wirbelschichtgerät (GPCG 1, Fa. GLATT) werden 800g KCI-Kristalle (0,3 - 0,8 mm) mit einer Sprühsuspension aus 373,3 g erfindungsgemäßer Dispersion nach Beispiel 12, 112 g Talkum, 0,95 g Antischaumemulsion und 412 g gereinigtem Wasser überzogen. Die Zulufttemperatur beträgt 30°C und der Sprühdruck an der Düse (Durchmesser 1,2 mm) 2,0 bar. Die Sprühdauer beträgt ca. 90 min.Nach dem 16-stündigem Trocknen bei Raumtemperatur erhält man gleichmäßige überzogene Kristalle.

Die Freigabe der Kaliumchloridkristalle wurde über 6 Stunden im Paddlegerät bei 100Upm in 900mL Wasser gemessen. Der Gehalt an Kaliumchlorid wurde potentiometrisch bestimmt.

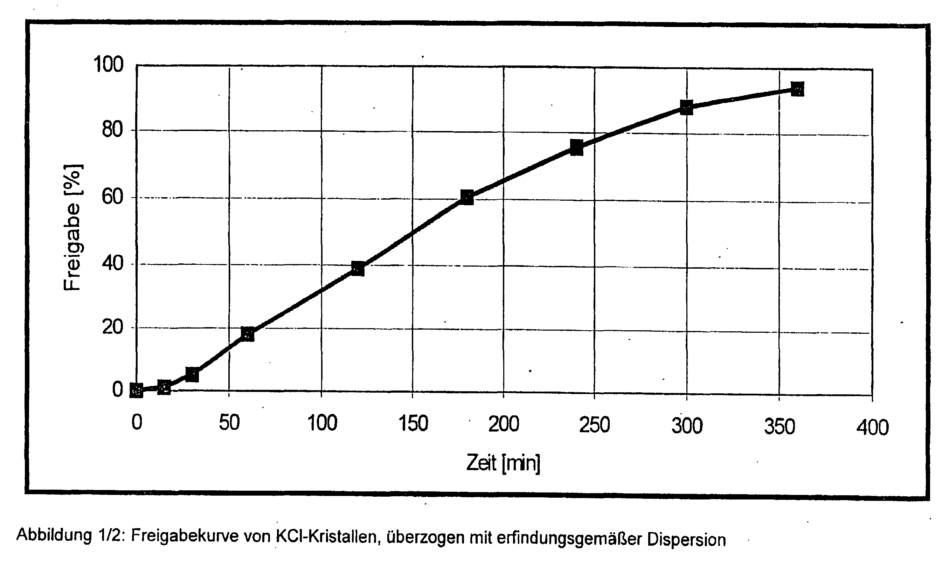

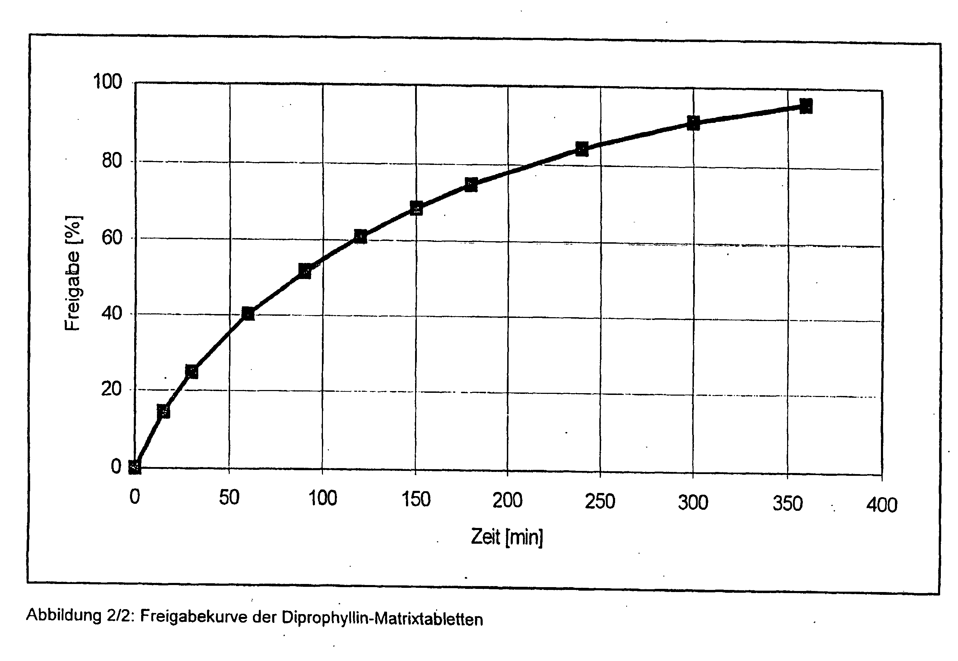

Das Freigabeprofil der mit erfindungsgemäßer Dispersion überzogenen Kaliumchloridkristalle lassen eine gleichmäßige Retadierung über 6h erkennen (siehe Abbildung 1/2).

### 20. Verwendung der Dispersion als Bindemittel :

Es werden Matrixtabletten mit einer Gesamtmasse von 600mg und einem Diprophyllingehalt von 150mg hergestellt. Für 1,2kg Matrixtabletten werden in der STEPHAN UM 12 300g Diprophyllin mit 400g Calciumhydrogenphosphatdihydrat (0,1 - 0,2mm) gemischt und anschließend mit erfindungsgemäßer Dispersion nach Beispiel 12 angefeuchtet. Nach Trocknung bei 40°C über 6h wird die Tablettenmasse über ein 1mm - Sieb gegeben, mit 12g Magnesiumstearat vermischt und auf einer KORSCH - Exzenter-Tablettenpresse mit 10kN verpresst. Die resultierenden Tabletten glänzen leicht, besitzen gute mechanische Festigkeit und zeigen eine gleichmäßige Freigabegeschwindigkeit über 6 - 7 Stunden.
Das Freigabeprofil der Matrixtabletten mit Diprophyllin zeigt ebenfalls eine gleichmäßige Retadierung (Abbildung 2/2). Die Wirkstofffreigabe wurde über 6 Stunden im Paddlegerät bei 50Upm in 900mL Wasser mit einem UV-VIS - Spektrometer Perkin-Elmer Lambda 20 bei 274nm bestimmt.

## Patentansprüche

1. Dispersion, geeignet zur Verwendung als Überzugs- und Bindemittel für Arzneiformen, mit einem Feststoffgehalt von 10 - 70 Gew.-%, enthaltend
a) 90 bis 99 Gew.-% eines Methacrylat-Copolymeren, das zu mindestens zu 90 Gew.-% aus (Meth)acrylat-Monomeren mit neutralen Resten besteht und eine Glastemperatur Tg von - 20 °C bis + 20 °C bestimmt nach der DSC-Methode, und
b) einen Emulgator,
**dadurch gekennzeichnet, daß**
als Emulgator b) 1 - 10 Gew.-% eines nichtionischen Emulgators mit einem HLB-Wert von 15, 2 bis 17,3 enthalten sind.

2. Dispersion nach Anspruch 1, **dadurch gekennzeichnet, daß** das Methacrylat-Copolymer zu 20 bis 50 Gew.-% aus Methylmethacrylat, 80 bis 50 Gew.-% Ethylacrylat und gegebenenfalls zu 0 bis 10 Gew.-% Methacrylsäure besteht.

3. Dispersion nach Anspruch 1 oder 2, **dadurch gekennzeichnet, daß** der nichtionische Emulgator ausgewählt wird aus Substanzgruppe der ethoxylierten Fettsäureester oder -ether, ethoxylierten Sorbitanether, ethoxylierten Alkylphenole, Glycerin- oder Zuckerester oder Wachsderivate.

4. Dispersion nach einem oder mehreren der Ansprüche 1 bis 3, **dadurch gekennzeichnet, daß** der nichtionische Emulgator ausgewählt wird aus der Gruppe Polyoxyethylenglycerinmonolaurat, Polyoxyethylenglycerinmonostearat, Polyoxyethylen-20-cetylstearat Polyoxyethylen-25-cetylstearat, Polyoxyethylen(25)oxypropylenmonostearat, Polyoxyethylen-20-sorbitanmonopalmitat, Polyoxyethylen-16-tert.oktylphenol, Polyoxyethylen-20-cetylether, Polyethylenglykol(1000)monocetylether, ethoxyliertes Rizinusöl, Polyoxyethylensorbitol-Wollwachs-Derivate, Polyoxyethylen(25)propylenglykolstearat, Polyoxyethylensorbitester Polyoxyethylen-25-cetylstearat, Polyoxyethylen-20-sorbitanmonopalmitat, Polyoxyethylen-16-tert.oktylphenol und Polyoxyethylen-20-cetylether

5. Verfahren zur Herstellung einer Dispersion.nach einem oder mehreren der Ansprüche 1 bis 4 in an sich bekannter Weise durch Emulsionspolymerisation.

6. Verwendung einer Dispersion nach einem oder mehreren der Ansprüche 1 bis 4 als Überzugs- oder Bindemittel zur Herstellung von Arzneimitteln.

7. Verwendung nach Anspruch 6, **dadurch gekennzeichnet, daß** die überzogenen oder gebundenen Arzneimittel einen der Wirkstoffe Morphin und dessen Derivate, Tramadol, Acetylsalicylsäure, Diclofenac, Indometacin, Lonazolac, Ibuprofen, Ketoprofen, Propyphenazon, Naproxen, Paracetamol, Flurbiprofen, Dimetinden, Chinidin, Metoprolol, Propranolol, Oxprenolol, Pindolol, Atenolol, Metoprolol, Disopyramid, Verapamil, Diltiazem, Gallopamil, Nifedipin, Nicardipin, Nisoldipin, Nimodipin, Amlodipin, Theophyllin, Salbutamol, Terbutalin, Ambroxol, Aminophyllin, Cholintheophyllinat, Pyridostigmin, Piretanid, Furosemid, Pentoxifyllin, Naftidrofuryl, Buflomedil, Xantinolnicotinat, Bencyclan, Allopurinol, Norephedrin, Clorphenamin lsosorbidmononitrat, Isosorbiddinitrat, Glyceroltrinitrat, Molsidomin, Bezafibrat, Fenofibrat, Gemfibrozil, Cerivastatin, Pravastatin, Fluvastatin, Lovastatin, Atorvastatin, Simvastatin, Xantinol, Metoclopramid, Amitriptylin, Dibenzepin, Venlafaxin, Thioridazin, Oxazepam, Lithium, Nitrofurantoin, pflanzliche Trockenextrakt, Ascorbinsäure und Kalium und deren pharmazeutisch verwendete Salze enthalten.

8. Arzneiform, enthaltend einen pharmazeutischen Wirkstoff, **dadurch gekennzeichnet, daß** der Wirkstoff mit einem Emulsionspolymerisat gebunden oder überzogen ist, das aus einer Dispersion nach einem oder mehreren der Ansprüche 1 bis 5 durch Trocknen erhalten wurde.

## Claims

1. Dispersion suitable for use as a coating agent and binder for pharmaceutical preparations, having a solids content of 10-70 wt.%, containing
a) 90 to 99 wt.% of a methacrylate copolymer which consists of at least 90 wt.% of (meth)acrylate monomers with neutral groups and has a glass transition temperature Tg of -20°C to +20°C determined by the DSC method, and
b) an emulsifier,
**characterised in that** it contains as the emulsifier b) 1-10 wt.% of a non-ionic emulsifier with an HLB value of 15.2 to 17.3.

2. Dispersion according to claim 1, **characterised in that** the methacrylate copolymer is made up of 20 to 50 wt.% of methyl methacrylate, 80-50 wt.% of ethyl acrylate and optionally 0-10 wt.% of methacrylic acid.

3. Dispersion according to claim 1 or 2, **characterised in that** the non-ionic emulsifier is selected from the group of substances comprising the ethoxylated fatty acid esters or ethers, ethoxylated sorbitan ethers, ethoxylated alkylphenols, glycerol or sugar esters or wax derivatives.

4. Dispersion according to one or more of claims 1 to 3, **characterised in that** the non-ionic emulsifier is selected from the group comprising polyoxyethyleneglycerol monolaurate, polyoxyethyleneglycerol monostearate, polyoxyethylene-20-cetylstearate, polyoxyethylene-25-cetylstearate, polyoxyethylene(25)oxypropylene monostearate, polyoxyethelene-20-sorbitan monopalmitate, polyoxyethylene-16-tert.octylphenol, polyoxyethylene-20-cetylether, polyethyleneglycol(1000)monocetylether, ethoxylated castor oil, polyoxethylenesorbitol wool grease derivatives, polyoxyethylene(25)propyleneglycol stearate, polyoxyethylenesorbitol ester, polyoxyethylene-25-cetylstearate, polyoxyethylene-20-sorbitan monopalmitate, polyoxyethylene-16-tert.octylphenol and polyoxyethylene-20-cetylether.

5. Process for preparing a dispersion according to one or more of claims 1 to 4 in a manner known per se by emulsion polymerisation.

6. Use of a dispersion according to one or more of claims 1 to 4 as a coating agent or binder for the preparation of pharmaceutical compositions.

7. Use according to claim 6, **characterised in that** the coated or bound pharmaceutical compositions contain one of the active substances morphine and the derivatives thereof, tramadol, acetylsalicylic acid, diclofenac, indomethacin, lonazolac, ibuprofen, ketoprofen, propyphenazone, naproxen, paracetamol, flurbiprofen, dimetinden, quinadine, metoprolol, propranolol, oxprenolol, pindolol, atenolol, disopyramide, verapamil, diltiazem, gallopamil, nifedipine, nicardipine, nisoldipine, nimodipine, amlodipine, theophylline, salbutamol, terbutaline, ambroxol, aminophylline, choline-theophyllinate, pyridostigmine, piretanide, furosemide, pentoxifylline, naftidrofuryl, buflomedil, xantinol nicotinate, bencyclan, allopurinol, norephedrin, chlorphenamine isosorbide mononitrate, isosorbide monomitrate, glycerol trinitrate, molsidomine, bezafibrate, fenofibrate, gemfibrozil, cerivastatin, pravastatin, fluvastatin, lovastatin, atorvaststatin, simvastatin, xantinol, metoclopramide, amitriptyline, dibenzepin, venlafaxine, thioridazine, oxazepam, lithium, nitrofurantoin, dry plant extract, ascorbic acid and potassium and the salts thereof which are used pharmaceutically.

8. Pharmaceutical composition containing a pharmaceutical active substance, **characterised in that** the active substance is bound to or coated with an emulsion polymer obtained from a dispersion according to one or more of claims 1 to 5 by drying.

## Revendications

1. Dispersion pour une utilisation comme agent de revêtement et comme liant, pour formes médicamenteuses, présentant un extrait sec de 10 - 70 % en poids, contenant
a) 90 à 99 % en poids d'un copolymère de méthacrylate, constitué à raison de 90 % en poids de monomères de (méth)acrylate avec des restes neutres et ayant une température de transition vitreuse Tg de - 20°C à +20 °C, déterminée par la méthode DSC,
et
b) un émulsionnant,
**caractérisée en ce que**
comme émulsionnant b), elle contient 1 à 10 % en poids d'un émulsionnant non ionique d'un indice HLB de 15,2 à 17,3.

2. Dispersion selon la revendication 1,
**caractérisée en ce que**
le copolymère de méthacrylate est constitué de 20 à 50 % en poids, de méthacrylate de méthyle, de 80 à 50 % en poids d'acrylate d'éthyle et, le cas échéant, de 0 à 10 % en poids d'acide méthacrylique.

3. Dispersion selon la revendication 1 ou 2,
**caractérisée en ce que**
l'émulsionnant non ionique est choisi dans le groupe de substances constitué des esters ou éthers d'acides gras éthoxylés, éthers de sorbitan éthoxylés, alkylphénols éthoxylés, esters de glycérol ou de sucre ou dérivés de cire.

4. Dispersion selon une ou plusieurs des revendications 1 à 3,
**caractérisée en ce que**
l'émulsionnant est choisi dans le groupe suivant :
monolaurate de polyoxyéthylèneglycérol,
monostéarate de polyoxyéthylèneglycérol,
20-cétylstéarate de polyoxyéthylène, 25-cétylstéarate de polyoxyéthylènen, monostéarate de polyoxyéthylène(25)oxypropylène, monopalmitate de polyoxyéthylène-20-sorbitane, 16-tert-octylphénol de polyoxyéthylène, 20-cétyléther de polyoxyéthylène, (1000)éther monocétylique de polyéthylène glycol, huile de ricin éthoxylée, dérivés de polyoxyéthylène-sorbitol-cire de laine, stéarate de polyoxyéthylène(25)propylèneglycol, ester de sorbitol de polyoxyéthylène, cétylstéarate 25-polyoxyéthylène-, 20-sorbitanemonopalmitate de polyoxyéthylène-, 16-tert-octylphénol de polyoxyéthylène, et 20-éther cétylique de polyoxyéthylène.

5. Procédé pour la préparation d'une dispersion selon une ou plusieurs des revendications 1 à 4, d'une manière connue en soi, par polymérisation en émulsion.

6. Utilisation d'une dispersion selon une ou plusieurs des revendications 1 à 4 comme agent de revêtement ou comme liant pour la préparation de médicaments.

7. Utilisation selon la revendication 6
**caractérisée en ce que**
les médicaments revêtus ou liés contiennent une des substances actives : morphine et ses dérivés, tramadol, acide acétylsalicylique, diclofénac, indométacine, lonazolac, ibuprofène, cétoprofène, propylphénazone, naproxène, paracétamol, flurbiprofène, dimétindène, quinidine, métoprolol, propranolol, oxprenolol, pindolol, aténolol, métoprolol, disopyramide, vérapamile, diltiazème, gallopamil, nifédipine, nicardipine, nisoldipine, nimodipine, amlodipine, théophylline, salbutanol, terbutaline, ambroxol, aminophylline, theophyllinate de choline, pyridostigmine, piréranide, furosamide, pentoxyfylline, naftidrofuryle, buflomédile, nicotinate de xantinol, bencyclane, allopurinol, noréphédrine, chlorophénamine, mononitrate d'isosorbide, dinitrate d'isosorbide, trinitrate de glycérol, molsidomine, bézafibrate, fénofibrate, gemfibrozil, cérivastatine, pravastatine, fluvastatine, lovastatine, atorvastatine, simvastatine, xantinol, métoclopramide, amitriptyline, dibenzépine, venlafaxine, thioridazine, oxazépame, lithium, nitrofurantoïne, extrait sec de plantes, acide ascorbique et potassium et leurs sels d'utilisation pharmaceutique.

8. Forme médicamenteuse contenant une substance active pharmaceutique,
**caractérisée en ce que**
la substance active est liée ou revêtue avec un polymérisat en émulsion qui a été obtenu par séchage à partir d'une dispersion selon une ou plusieurs des revendications 1 à 5.
